# EUROPEAN PATENT SPECIFICATION

(11) **EP 2 985 037 B1**
(45) Date of publication and mention of the grant of the patent: **21.06.2017**
(21) Application number: 15177507.9
(22) Date of filing: 20.07.2015
(51) Int. Cl.: A61K 31/16, A61K 9/00, A61K 9/14, A61K 9/20, A61K 31/05, A61K 31/164, A61K 31/352, A61K 31/7048, A61K 31/7068, A61P 25/28, A61K 45/06

(54) **A pharmaceutical composition comprising palmitoylethanolamid and cytidine-diphosphocholine**
Pharmazeutische Zusammensetzung enthaltend Palmitoylethanolamid und Citicoline.
Composition pharmaceutique comprenant le palmitoyethanolamide et le citicoline

(30) Priority: 13.08.2014 IT MI20141495
(43) Date of publication of application: 17.02.2016
(73) Proprietor: EPITECH GROUP S.p.A., 20144 Milano (MI) (IT)
(72) Inventor: DELLA VALLE, Francesco, I-20144 MILANO (IT); DELLA VALLE, Maria Federica, I-20144 MILANO (IT); MARCOLONGO, Gabriele, I-20144 MILANO (IT); CUZZOCREA, Salvatore, I-20144 MILANO (IT)
(74) Representative: Long, Giorgio

(56) References cited:
- EP-A1- 2 444 078
- WO-A1-2011/027373
- DE-A1-102005 056 558
- LOZANO ET AL: "CDP-choline in the treatment of cranio-encephalic traumata", JOURNAL OF NEUROLOGICAL SCIENCES, ELSEVIER SCIENTIFIC PUBLISHING CO, AMSTERDAM, NL, vol. 103, 1 July 1991 (1991-07-01), pages 43-47, XP024299590, ISSN: 0022-510X, DOI: 10.1016/0022-510X(91)90008-U [retrieved on 1991-07-01]
- VILLA R F ET AL: "Effect of CDP-choline treatment on mitochondrial and synaptosomal protein composition in different brain regions during aging", INTERNATIONAL JOURNAL OF DEVELOPMENTAL NEUROSCIENCE, PERGAMON, OXFORD, GB, vol. 11, no. 1, 1 February 1993 (1993-02-01), pages 83-93, XP024392045, ISSN: 0736-5748, DOI: 10.1016/0736-5748(93)90037-E [retrieved on 1993-02-01]

## Description

### Technical field of the invention

The object of the present invention is a pharmaceutical composition for use in humans or animals containing N-palmitoylethanolamide and Cytidine-diphosphocholine for the treatment of pathologies of the Central Nervous System of a traumatic, vascular, degenerative nature, associated with neurodegeneration.

### State of the art

The neurodegeneration of cerebral neuron circuits constitutes a highly relevant process in the occurrence of neurocognitive and behavioural disorders following traumatic or vascular events with encephalic localization or following degenerative processes generally associated with aging. The most recent literature increasingly considers neurodegeneration to be a consequence of neuroinflammation processes, either acute or chronic, primarily with encephalic localization or resulting from peripheral chronic inflammation processes that are not adequately controlled, even low-grade inflammation, continuing for an excessively long time. It has also now been ascertained that an active contributor to neurodegeneration is also oxidative stress that is generated in neuroinflammation sites as a

consequence of activation of specific processes now widely investigated by international neuroscientists. In neuroinflammation mechanisms, both peripheral and central, an important role is played by so-called non-neuronal cells, in part belonging to the Immune System (mast-cell, microglia, astrocyte, oligodendrocyte) that from a physiologically neuroprotective role, following specific acute and/or chronic noxae, can take on a highly aggressive role for the neuron of certain areas of the brain until there is a profound alteration of synaptogenesis, neuronal death and slowing of compensatory neurogenesis. Neurodegeneration processes begin with the perturbation of neuron cellular homeostasis and in particular with damage to the phospholipid components constituting the cellular membranes due to slowing of phospholipid synthesis, and consequent alteration of the membrane fluidity of the neuronal cells.

For some time it has been known that palmitoylethanolamide, an endogenous lipid of N-acylethanolamidic nature produced *on demand* in the case of cellular damage, is able to modulate, in an inhibitory manner, both the hyperdegranulation of the mastocyte and the hyper-activation of the microglia and of the astrocyte thus proving capable, if administered in pharmaceutical form such as to ensure the bioavailability thereof at the level of the target cells, of controlling both the peripheral and central neuroinflammation processes. In particular, at the preclinical level, palmitoylethanolamide has demonstrated a substantial effect, at the optimal dose of 10 mg/kg, in the stroke model from ligation of the middle cerebral artery or in the cranial trauma model or in that of dementia from damage due to beta amyloid; at the clinical level, many studies have demonstrated the ability of palmitoylethanolamide, administered orally in a suitable physical form - for example micronized and/or ultramicronized - to control the neuroinflammation processes like in Amyotrophic Lateral Sclerosis, in Multiple Sclerosis, in post-stroke functional recovery (see for example, European Patent 2475352, application number ep09741462.7, filed on 07.09.09). Cytidine-diphosphocholine (CDP-Choline or Citicoline), a molecule that has been used for many years in clinics in the treatment of cognitive and behavioural disorders in the elderly, has proved capable, at the dose of 500 mg/kg, of reducing the parenchymal cerebral damage in the stroke model in animals from experiment [Dávalos A, Secades J.Citicoline preclinical and clinical update 2009-2010. Stroke. 2011 Jan;42(1 Suppl):S36-9].

The recognised mechanism of action for such molecules essentially consists of an activation of the synthesis of an important membrane phospholipid, phosphatidylcholine.

### Summary of the invention

The inventors of the present patent have surprisingly discovered that the association between palmitoylethanolamide, in a micronized or ultramicronized form, and Cytidine-diphosphocholine is capable of determining a highly synergic effect between the two molecules, an effect that is particularly evident in the protection from parenchymal cerebral damage, after ischemic suffering determined by ligation of the middle cerebral artery that induces firstly a state of acute neuroinflammation and then consequent neurodegeneration.

They have also discovered that the addition of a molecule with antioxidant activity to the association between palmitoylethanolamide and cytidine-diphosphocholine - possibly co-micronized with palmitoylethanolamide - further enhances the synergy between the two main components of the invention.

The object of the present invention is therefore a pharmaceutical composition for oral and/or parenteral use comprising palmitoylethanolamide (PEA), alternatively in a non-micronized form (non micronized PEA), or in a micronized form (PEA-m), or in an ultramicronized form (PEA-um), and cytidine-diphosphocholine.

A further object of the invention is a pharmaceutical composition comprising palmitoylethanolamide (PEA) as defined above, cytidine-diphosphocholine and anti-oxidant molecules of the family of polyphenols (for example, polydatin, resveratrol, luteolin, quercetin, rutin, etc.), α-lipoic acid and/or acetylcystein.

The invention is defined in the attached claims.

### Detailed description of the invention

The invention concerns a pharmaceutical composition comprising palmitoylethanolamide (PEA), alternatively in a non-micronized form (non micronized PEA), or in a micronized form (PEA-m), or in an ultramicronized form (PEA-um), and Cytidine-diphosphocholine.

The palmitoylethanolamide can be synthesised as described in example no. 25 of patent US 5,990,170.

The non micronized PEA can be obtained by finely grinding the product coming from synthesis; a product can be obtained with a particle size ranging between 50.0 and 100.0 µm.

The PEA-m can be obtained as described in patent US 6,548,550 B1 and has a particle size ranging between 2.0 and 10.0 µm.

The PEA-um can be obtained as described in application PCT published with no. WO 2011/027373 A1 and has a particle size ranging between 0.8 and 6.0 µm.

More indication on said forms of PEA are to be found in the aforementioned patent publications, the content of which relative to the characterisation of the product is incorporated here for reference.

Pharmaceutical grade Cytidine-diphosphocholine is a commercial product.

The pharmaceutical composition of the invention comprises palmitoylethanolamide in weight percentages ranging between 20 and 35%, Cytidine-diphosphocholine in weight percentages between 20 and 55% and one or more compounds with antioxidant activity in total weight percentages ranging between 0 and 20%.

The compound with antioxidant activity is preferably selected from the group comprising polyphenols, alpha-lipoic acid (or thioctic acid) and L-acetylcysteine.

When the antioxidant compound is or comprises a polyphenol, it is preferably selected from polydatin, resveratrol, luteolin, quercetin and rutin.

The molecules with antioxidant activity can also be co-micronized or co-ultramicronized with the palmitoylethanolamide according to the teachings described in patent US 6,548,550 B1.

The anti-oxidant compounds are commercial products.

The inventive composition can also contain pharmaceutically acceptable excipients and additives, selected as a function of the preselected pharmaceutical form.

The pathologies that can be treated with the composition object of the present invention comprise:
- Neurocognitive and/or behavioural disorders following cranioencephalic injuries
- Neurocognitive and/or behavioural disorders following vascular, of anoxic and/or hemorrhagic nature, events, with encephalic localization
- Neurocognitive and/or behavioural disorders associated to neurodegenerative states of a toxic, infectious, dysmetabolic nature with encephalic localization, also associated to aging

The pharmaceutical composition according to the present invention can be formulated for oral, buccal, intragastric or parenteral administration.

For oral administration, the pharmaceutical compositions can, for example, be in the form of tablets or capsules prepared in the conventional manner with the pharmaceutically acceptable excipients as binding agents (for example pregelatinized corn starch, polyvinilpyrrolidone or methylcellulose hydroxypropyl); filler agents (for example lactose, microcrystalline cellulose or calcium hydrogen phosphate); lubricants (for example magnesium stearate, talc or silica); disintegrants (for example potato starch or sodium starch glycolate); or inhibiting agents (for example sodium lauryl sulphate). The tablets can be coated with the methods well known in the field. The liquid preparations for oral administration can, for example, be in the form of solutions, syrups or suspensions or they can be lyophilized products to be reconstituted, before use, with water or other suitable carriers. Such liquid preparations can be prepared through the conventional methods with the pharmaceutically acceptable additives such as suspension agents (for example sorbitol syrup, cellulose derivatives or edible hydrogenated fats); emulsifying agents (for example lecithin or acacia); non-aqueous carriers (for example almond oil, oily esters, ethyl alcohol or fractioned vegetable oils); and preservatives (for example methyl- or propyl-p-hydroxybenzoates or sorbic acid). The preparation can also suitably contain flavourings, dyes and sweeteners.

The preparations for oral administration can be formulated in a suitable manner to allow the controlled release of the active ingredient; they can also be formulated for administration directly into the stomach through nasogastric intubation.

For buccal administration, the compositions can be in the form of granulates, tablets or pills formulated in the conventional manner, suitable for absorption at the level of the buccal mucosa. Typically buccal formulations are tablets or granulates for sub-lingual administration.

According to the present invention, the compounds can also be formulated for parenteral administrations and in particular intramuscularly or endovenously suspended and/or dissolved in suitable liquid media.

In addition to the compositions described above, the compounds can also be formulated as depot preparations. Such long-acting formulations can be administered by implantation (for example subcutaneously, transcutaneously or intramuscularly) or by intramuscular injection. Therefore, for example, the compounds, according to the present invention, can be formulated with appropriate polymeric or hydrophobic materials (for example in the form of an emulsion in a suitable oil) or ion exchange resins or as minimally soluble derivatives, for example as minimally soluble salt.

According to the present invention the dose of compounds proposed for administration to a human (with body weight of about 70 Kg) ranges from 10 mg to 1 g and, preferably from 100 mg to 500 mg of the active ingredients per dose unit. The dose unit can be administered, for example, from 1 to 4 times per day. The dose will depend on the preselected method of administration. It should be considered that it could be necessary to make continuous variations of the dosage according to the age and weight of the patient and also the severity of the clinical condition to be treated. The exact dose and the method of administration will finally be at the discretion of the treating physician or of the veterinarian.

The formulations described above can be prepared according to conventional methods, like those described in Remington's Pharmaceutical Sciences Handbook, Mack Pub. Co., N.Y., USA.

### EXPERIMENTAL PART

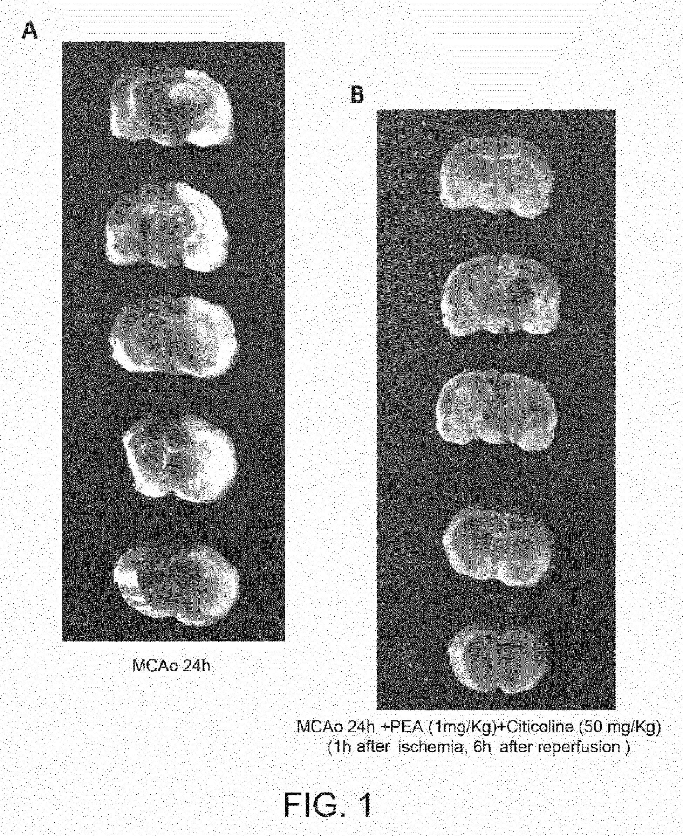

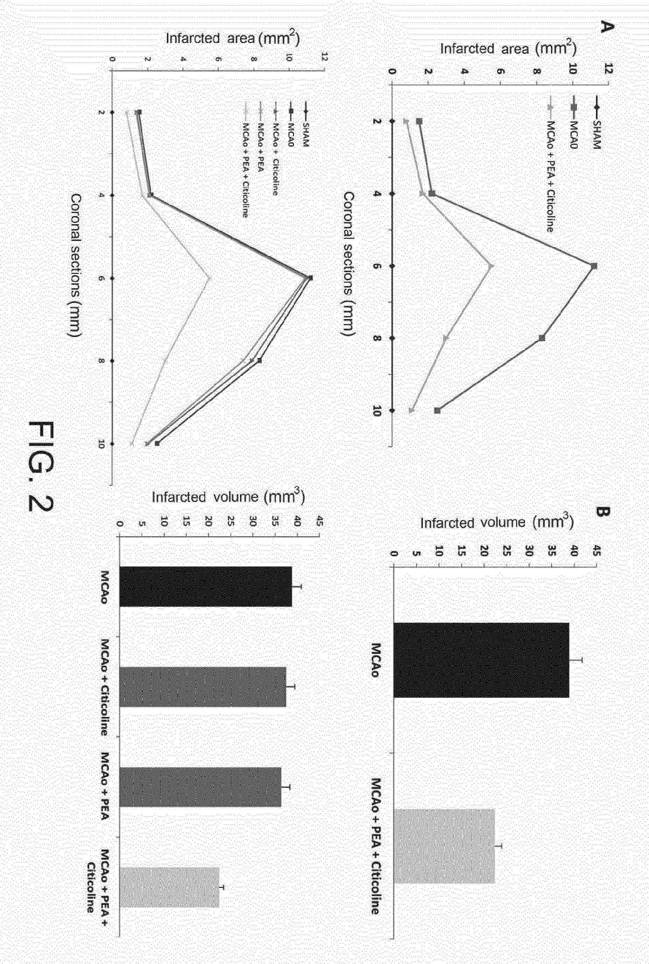

In order to demonstrate the synergic effect of the two substances, the middle cerebral artery in rats was occluded by ligation thereof according to the method described by Ahmad et al 2012 [Ahmad A, Genovese T, Impellizzeri D, Crupi R, Velardi E, Marino A, Esposito E, Cuzzocrea S. Reduction of ischemic brain injury by administration of palmitoylethanolamide after transient middle cerebral artery occlusion in rats. Brain Res. 2012 Oct 5;1477:45-58]. Using such a method the maximum ineffective dose of the two substances, ultramicronized Palmitoylethanolamide and cytidine-diphosphocholine, administered separately orally suspended in carboxymethyl cellulose at 1.5% was initially sought. In the maximum ineffective doses found, the two substances were then mixed and administered together again suspended in CMC at 1.5%. The results obtained are collected in the following table 1 and the data of the synergic association between PEA and Citicoline are presented in figure 1 and figure 2.

**Table 1**

| Treatment | Administered dose mg/Kg | Infarction Area in mm² | % Infarction Area |
|---|---|---|---|
| | | | |
| only CMC 1.55 % | | 38,5 | 100,00 |
| only PEA-um in CMC 1.5% | 1 mg/Kg | 38,2 | 99,22 |
| only Citicoline in CMC 1.5% | 50 mg/Kg | 38,3 | 99,48 |
| PEA-um + Citicoline in CMC 1,5% | 1 mg/Kg + 50 mg/Kg | 22,4 | 58,18 |

Figure 1 shows images of coronal sections after occlusion of the middle cerebral artery (MCAo) in the absence, panel A, or in the presence of the combined treatment described above (panel B). Figure 2 highlights the detected measurements of the infarctual area (A) and volume (B) in the absence or in the presence of the treatment.
The invention will now be described further by some examples of formulation, given as examples and not limiting the scope of protection of the invention as defined by the attached claims.

### Examples of formulation

### Example A Tablets for oral use

Each tablet contains:
- PEA-um mg 150,0
- Citicoline mg 300,0
- Microcrystalline cellulose mg 80,0
- Croscarmellose sodium mg 45,0
- Polyvinylpyrrolidone mg 10,0
- Magnesium stearate mg 4,0

### Example B Tablets for oral use

Each tablet contains:
- PEA-m mg 200,0
- Citicoline mg 300,0
- Microcrystalline cellulose mg 90,0
- Croscarmellose sodium mg 65,0
- Polyvinylpyrrolidone mg 15,0
- Magnesium stearate mg 4,0

### Example C Tablets for oral use

Each tablet contains:
- PEA-um (co-ultramicronized
   with Luteolin) mg 200,0
- Luteolin (co-ultramicronized
   with PEA-m) mg 20,0
- Citicoline mg 300,0
- Microcrystalline cellulose mg 90,0
- Croscarmellose sodium mg 65,0
- Polyvinylpyrrolidone mg 15,0
- Magnesium stearate mg 4,0

### Example D Tablets for oral use

Each tablet contains:
- PEA-um (co-ultramicronized
   with resveratrol) mg 200,0
- Resveratrol (co-ultramicronized
   with PEA) mg 25,0
- Citicoline mg 300,0
- Microcrystalline cellulose mg 90,0
- Croscarmellose sodium mg 65,0
- Polyvinylpyrrolidone mg 15,0
- Magnesium stearate mg 4,0

### Example and Tablets for oral use

Each tablet contains:
- PEA-um mg 200,0
- Acetylcysteine mg 50,0
- Citicoline mg 300,0
- Microcrystalline cellulose mg 90,0
- Croscarmellose sodium mg 65,0
- Polyvinylpyrrolidone mg 15,0
- Magnesium stearate mg 4,0

### Example F Tablets for oral use

Each tablet contains:
- PEA-m mg 150,0
- α-lipoic acid mg 100,0
- Citicoline mg 200,0
- Microcrystalline cellulose mg 100,0
- Croscarmellose sodium mg 80,0
- Polyvinylpyrrolidone mg 10,0
- Magnesium stearate mg 5,0

### Example G microgranules for sub-lingual use

Each packet of microgranules contains:
- PEA-um mg 300,0
- Acetylcysteine mg 100,0
- Citicoline mg 300,0
- Sorbitol powder mg 380,0
- Sucrose palmitate mg 15,0
- Polysorbate 80 (vegetable) mg 5,0.

### Example H suspension for injective use

Each 5 ml vial for endovenous use contains:
- PEA-um mg 400,0
- Acetylcysteine mg 100,0
- Citicoline mg 600,0
- Injectable surfactant agent mg 10,0
- Pyrogen-free double-distilled water to make up 5,0 ml

## Claims

1. A pharmaceutical composition comprising palmitoylethanolamide (PEA) and cytidine-diphosphocholine (CDP-choline or citicoline).

2. The pharmaceutical composition according to claim 1, wherein said palmitoylethanolamide is in a non-micronized form with a particle size ranging between 50.0 and 100.0 µm, in a micronized form (PEA-m) with a particle size ranging between 2.0 and 10.0 µm, or in an ultramicronized form (PEA-um) with a particle size ranging between 0.8 and 6.0 µm, or in a mixture of such forms.

3. The pharmaceutical composition according to claim 1 or 2, wherein said cytidine-diphosphocholine is in a finely pulverized form.

4. The pharmaceutical composition according to any of the claims 1 to 3, comprising palmitoylethanolamide in weight percentages ranging between 20 and 35%, cytidine-diphosphocholine in weight percentages between 20 and 55%, and one or more compounds with antioxidant activity in total weight percentages ranging between 0 and 20%.

5. The pharmaceutical composition according to claim 4, wherein the compound with antioxidant activity is selected from the group comprising polyphenols, alpha-lipoic acid, and L-acetylcysteine.

6. The pharmaceutical composition according to claim 5, wherein, when the antioxidant compound is or comprises a polyphenol, it is preferably selected between polydatin, resveratrol, luteolin, quercetin, and rutin.

7. The pharmaceutical composition according to claim 5 or 6, wherein the compound with antioxidant activity is co-micronized or co-ultramicronized with palmitoylethanolamide.

8. The pharmaceutical composition according to any of the claims 1 to 7, wherein said composition is for oral or buccal or intragastric or parenteral use.

9. The pharmaceutical composition according to any of the claims 1 to 8, for use in the treatment of a disease selected from:
- neurocognitive and/or behavioral disorders following *cranioencephalic injuries,*
- neurocognitive and/or behavioral disorders following vascular, of anoxic and/or hemorrhagic nature, events, with encephalic localization,
- neurocognitive and/or behavioral disorders associated to neurodegenerative states of a toxic, infectious, dysmetabolic nature with encephalic localization, also associated to aging.

## Patentansprüche

1. Pharmazeutische Zusammensetzung, umfassend Palmitoylethanolamid (PEA) und Cytidindiphosphocholin (CDP-Cholin oder Citicolin).

2. Pharmazeutische Zusammensetzung nach Anspruch 1, wobei das Palmitoylethanolamid in einer nicht-mikronisierten Form mit einer Partikelgröße im Bereich von zwischen 50,0 und 100,0 µm, in einer mikronisierten Form (PEA-m) mit einer Partikelgröße im Bereich von zwischen 2,0 und 10,0 µm, oder in einer ultra-mikronisierten Form (PEA-um) mit einer Partikelgröße im Bereich von zwischen 0,8 und 6,0 µm, oder in einem Gemisch solcher Formen, vorliegt.

3. Pharmazeutische Zusammensetzung nach Anspruch 1 oder 2, wobei das Cytidindiphosphocholin in einer fein pulverisierten Form vorliegt.

4. Pharmazeutische Zusammensetzung nach einem der Ansprüche 1 bis 3, umfassend Palmitoylethanolamid in Gewichtsanteilen im Bereich von zwischen 20 und 35%, Cytidindiphosphocholin in Gewichtsanteilen von zwischen 20 und 55%, und eine oder mehrere Verbindungen mit antioxidativer Aktivität in Gesamtgewichtsanteilen im Bereich von zwischen 0 und 20%.

5. Pharmazeutische Zusammensetzung nach Anspruch 4, wobei die Verbindung mit antioxidativer Aktivität ausgewählt ist aus der Gruppe umfassend Polyphenole, Alpha-Liponsäure und L-Acetylcystein.

6. Pharmazeutische Zusammensetzung nach Anspruch 5, wobei die pharmazeutische Zusammensetzung, wenn die antioxidative Verbindung ein Polyphenol ist oder umfasst, vorzugsweise ausgewählt ist aus Polydatin, Resveratrol, Luteolin, Quercetin und Rutin.

7. Pharmazeutische Zusammensetzung nach Anspruch 5 oder 6, wobei die Verbindung mit antioxidativer Aktivität mit Palmitoylethanolamid co-mikronisiert oder co-ultramikronisiert ist.

8. Pharmazeutische Zusammensetzung nach einem der Ansprüche 1 bis 7, wobei die Zusammensetzung zur oralen oder bukkalen oder intragastrischen oder parenteralen Verwendung ist.

9. Pharmazeutische Zusammensetzung nach einem der Ansprüche 1 bis 8 zur Verwendung bei der Behandlung einer Krankheit, ausgewählt aus:
- neurokognitiven Störungen und/oder Verhaltensstörungen infolge von kranioenzephalischen Verletzungen
- neurokognitiven Störungen und/oder Verhaltensstörungen infolge von vaskulären Ereignissen anoxischer oder hämorrhagischer Natur, mit enzephalischer Lokalisierung,
- neurokognitiven Störungen und/oder Verhaltensstörungen im Zusammenhang mit neurodegenerativen Zuständen toxischer, infektiöser, dysmetabolischer Natur mit enzephalischer Lokalisierung, auch im Zusammenhang mit Altern.

## Revendications

1. Composition pharmaceutique comprenant du palmitoyléthanolamide (PEA) et de la cytidine-diphosphocholine (CDP-choline ou citicoline).

2. Composition pharmaceutique selon la revendication 1, dans laquelle ledit palmitoyléthanolamide est sous une forme non micronisée ayant une taille de particule située entre 50,0 et 100,0 µm, sous une forme micronisée (PEA-m) ayant une taille de particule située entre 2,0 et 10,0 µm, ou sous une forme ultramicronisée (PEA-um) ayant une taille de particule située entre 0,8 et 6,0 µm, ou dans un mélange de ces formes.

3. Composition pharmaceutique selon la revendication 1 ou 2, dans laquelle ladite cytidine-diphosphocholine est sous une forme finement pulvérisée.

4. Composition pharmaceutique selon l'une quelconque des revendications 1 à 3, comprenant du palmitoyléthanolamide en des pourcentages en poids situés entre 20 et 35 %, de la cytidine-diphosphocholine en des pourcentages en poids situés entre 20 et 55 %, et un ou plusieurs composés ayant une activité antioxydante en des pourcentages en poids totaux situés entre 0 et 20 %.

5. Composition pharmaceutique selon la revendication 4, dans laquelle le composé ayant une activité antioxydante est choisi dans le groupe comprenant les polyphénols, l'acide alpha-lipoïque, et la L-acétyl-cystéine.

6. Composition pharmaceutique selon la revendication 5, dans laquelle, quand le composé antioxydant est ou comprend un polyphénol, il est de préférence choisi entre la polydatine, le resvératrol, la lutéoline, la quercétine, et la rutine.

7. Composition pharmaceutique selon la revendication 5 ou 6, dans laquelle le composé ayant une activité antioxydante est co-micronisé ou co-ultramicronisé avec le palmitoyléthanolamide.

8. Composition pharmaceutique selon l'une quelconque des revendications 1 à 7, dans laquelle ladite composition est destinée à une utilisation orale ou buccale ou intra-gastrique ou parentérale.

9. Composition pharmaceutique selon l'une quelconque des revendications 1 à 8, pour son utilisation dans le traitement d'une maladie choisie parmi :
- les troubles neurocognitifs et/ou du comportement faisant suite à des lésions cranio-encéphaliques,
- les troubles neurocognitifs et/ou du comportement faisant suite à des événements vasculaires, de nature anoxique et/ou hémorragique, avec localisation encéphalique,
- les troubles neurocognitifs et/ou du comportement associés aux états neurodégénératifs de nature toxique, infectieuse, dysmétabolique avec localisation encéphalique, également associés au vieillissement.
